# EUROPEAN PATENT SPECIFICATION

(11) **EP 2 743 703 B1**
(45) Date of publication and mention of the grant of the patent: **18.05.2022**
(21) Application number: 13196406.6
(22) Date of filing: 10.12.2013
(51) Int. Cl.: G01N 35/00, G01N 35/04, G01N 33/566, G06M 1/00

(54) **Method for holding multiple types of diagnostic test consumables in a random access single container**
Verfahren zum Halten mehrerer Arten von Diagnosetestverbrauchsartikeln in einem einzelnen Direktzugriffsbehälter
Procédé pour supporter de multiples types de produits consommables de test diagnostique dans un récipient unique à accès aléatoire

(30) Priority: 11.12.2012 US 201213710857; 08.03.2013 US 201313790751
(43) Date of publication of application: 18.06.2014
(73) Proprietor: ORTHO-CLINICAL DIAGNOSTICS, INC., Raritan, NJ 08869 (US)
(72) Inventor: Jorgensen, Jens H., Rochester, NY New York 14612 (US); Wycallis, Joseph, Pittsford, NY New York 14534 (US)
(74) Representative: Schweizer, Christiane

(56) References cited:
- EP-A1- 2 295 982
- EP-A2- 0 576 291
- US-A1- 2008 178 653

## Description

### FIELD OF THE INVENTION

The application relates to the field of immunodiagnostic testing using an automated analyzer and in particular to a method and device for holding a selection of immunological test elements or consumables in one or more containers attached to or placed into the analyzer and providing random access to any test element therein. The container is conveniently in the form of a sleeve, or rack that may be placed in a drawer adjacent and connected to the loading area of the analyzer. Such container can hold multiple types of test elements in compartments or slots. Through sensing of a test element position in its slot, the detection mechanism of the invention provides for random access to multiple types of test elements in any sleeve and within a single sleeve, and further provides efficient inventory control. Thus the method increases the number of test element types that may be loaded onto an analyzer while maintaining fast determination of inventory.

### BACKGROUND OF THE INVENTION

Immunological agglutination reactions are currently used for identifying various kinds of blood types as well as for detecting various kinds of antibodies and antigens in blood samples and other aqueous solutions. In such procedures, a sample of red blood cells is mixed with serum or plasma in a consumable device such as a test tubes, microplates or in the method knows in the art as column agglutination technology (CAT), a card or cassette tube configuration, wherein the mixture is incubated and then centrifuged. Various reactions then occur or do not occur depending on, for example, the blood types of the red blood cells or whether certain antibodies are present within the blood sample. These reactions manifest themselves as clumps of cells or as particles with antigens or antibodies on their surfaces, referred to as agglutinates. The failure of any agglutinates to appear indicates no reaction has occurred, while the presence of agglutinates, depending on the size and amount of the clumps formed, indicates the presence of a reaction and the level of concentration of cells or antibodies in the sample and reaction strength.

As described, for example, in U.S. Patent No. 5,512,432 to LaPierre et al., an agglutination test method has been developed and successfully commercialized, which method employs gel or glass bead microparticles contained within a small column, referred to as a microcolumn or a microtube. The said microcolumn or microtube is arranged as one of a plurality of columns formed in a transparent card or cassette format wherein multiple such tubes containing reagents are molded into a single consumable. A reagent, such as anti-A, is dispensed in a diluent in the microcolumns of the card or cassette and test red blood cells are placed in the reaction chamber above the column. The column, as part of the entire card or cassette, is then centrifuged. The centrifugation accelerates the reaction, if any, between the red blood cells and the reagent, and also urges any cells toward the bottom of the column. In the meantime, the glass beads or the gel material acts as a filter, and resists or impedes downward movement of the particles in the column. As a result, the nature and distribution of the particles in the microcolumn provides a visual indication of whether any agglutination reaction has occurred, and if such a reaction has occurred, the strength of the reaction based on the relative position of the agglutinates in the column. If no agglutination reaction has occurred, then all or virtually all of the red blood cells in the microtube will pass downward during the centrifugation procedure, to the bottom of the column in the form of a pellet. Conversely and if there is a strong reaction between the reagent and the red blood cells, then virtually all of the red blood cells will agglutinate, and large groupings will form at the top of the microtube above the gel or bead matrix in that the matrix is sized not to let these clumps pass through. Reactions falling between these latter two extremes are possible in which some but not all of the red blood cells will have agglutinated. The percentage of red blood cells that agglutinate and the size of the agglutinated particles each have a relationship with the strength of the reaction. Following the centrifugation process and after all processing steps have been completed, the microtube is visually examined by either a human operator or by machine vision such as a CCD camera for imaging the resulting reaction between the red blood cells and the reagent which is then classified. The reaction is classified as being either positive or negative, and if positive, the reaction is further classified into one of four classes depending on the strength of the reaction.

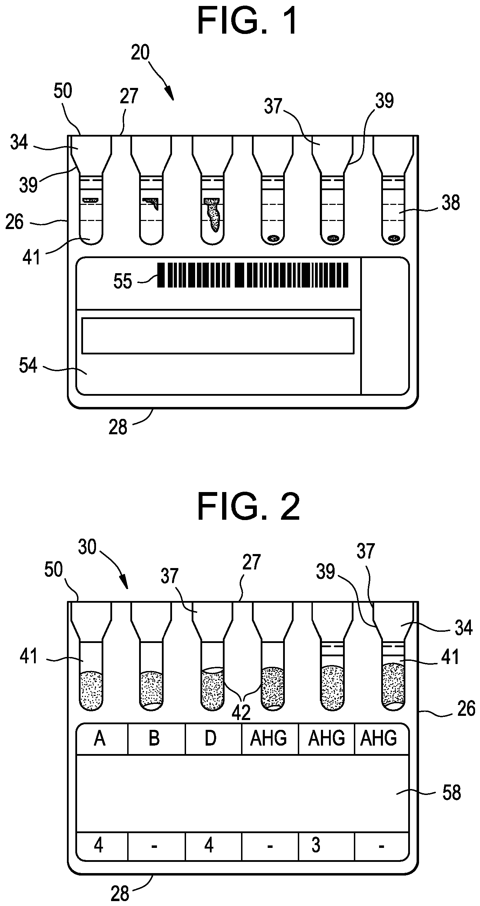

Currently, clinical immunohematology utilizes so-called gel cards and/or glass bead cassettes which are known consumable test elements and employ a plurality of microtubes for purposes of creating agglutination reactions as described above for blood grouping, blood typing, antigen or antibody detection and other related applications and uses. Thus, multiple types of test elements are known for the various blood grouping, typing and antigen antibody tests. These consumable test elements commonly include a planar substrate that supports a plurality of transparent columns or microtubes, each of the columns containing a quantity of an inert material, such as the aforementioned gel material or glass beads, respectively, that is coated with an antigen or antibody or material or is provided with a carrier-bound antibody or antigen, each of the foregoing being provided by the manufacturer. A pierceable wrap completes the assembly of the test element. This wrap which may be for example in the form of an adhesively or otherwise -attached foil wrap, covers the top side of the test element to cover the contents of each column. This same foil wrap conveniently provides a reflective surface which is utilized in the method of the instant invention as detailed hereinbelow. Once the covering wrap is pierced, aliquots of patient sample and possibly reagents (e.g., if reagents are not first added by the manufacturer or additional reagents, depending on the test) can be added to the columns, either manually or using automated apparatus. The test element thus containing patient sample (e.g., red blood cells and sera) is then incubated and following incubation, the test element is spun down by centrifugation, as noted above, in order to accelerate an agglutination reaction that can be graded either based on the position of agglutinates within each transparent column of the test element or cassette or due to a lack of agglutination based on the cells settling at the bottom of the test column. As shown in Figs 1 & 2, also present on the test element 20, 30 is typically located a barcode 55 bearing information identifying the reagents for the immunohematologic test type for that test element. Other barcode information on the test element can include shelf expiration, lot number, and the sequence of that test element within a given manufacturer's lot, among any other indicating information as desired by the manufacturer.

A number of automated or semi-automated apparatus, such as those manufactured by Ortho-Clinical Diagnostics, Inc., DiaMed A.G., Bio-Rad, and Grifols, are known that utilize a plurality of test elements in the form of gel cards or bead cassettes, such as those manufactured and sold by Micro-Typing Systems, Inc., DiaMed A.G., and BioRad, among others. Currently, test elements for a single immunological assay type are obtained from the manufacturer arranged in containers such as boxes or sleeves having multiple such cards or cassettes in separate slots. These boxes or sleeves conveniently fit in lanes of a slide tray in a drawer which is part of the analyzer. Depending on analyzer type, size and capacity, the slide tray in the drawer of an analyzer may accommodate from five (5) to twelve (12) such lanes separated by rails, permitting from five (5) to twelve (12) sleeves to be accommodated in an analyzer. Each container (sleeve) may contain for example twenty (20) cards or cassettes. This physical space limitation for sleeves and sleeve capacity restricts the types of immunological test element types to a maximum of twelve (12), one type per sleeve. However, there are currently about fifteen (15) to twenty (20) different test element (cards or cassettes) types available for use in blood analysis testing, for example including various manufacture-available ABO blood-type and blood antibody-type test element cards/cassette types. Thus the requirement for operator intervention to insert and exchange specific cards upon physician order is high. The operator or technician using the apparatus must therefore load the appropriate sleeve containing the desired cards or cassettes, which requires opening the card/cassette loading area (CCLA) of the apparatus and manually inserting into a slot within the sleeve the one or more desired cards or cassettes for the appropriate immunological test(s). Such manual interaction by the operator with the analyzer requiring opening the analyzer drawer to access the sleeves and changing the test element necessarily interrupts the blood testing process and delays results.

As described, each of the consumable test elements includes a top side adhesive wrap or other protective sealing cover. This wrap or cover conveniently comprises a protective sealing wrap such as a foil wrap which covers the microcolumns and forms a seal relative to the contents of the microcolumns further preventing microcolumn contents from drying out or degrading. To allow for inventory control, analyzers made by the above-mentioned companies are equipped with software permitting detection functionality to determine which consumable or test element (card or cassette) positions are in fact loaded with a consumable test element and of which type. In one aspect of the invention, using a processor an optical sensor measures and thereby detects the reflective difference between the presence and absence of the foil wrapped consumable test element in a position. Such an optical sensor can be for example an optical proximity sensor. An algorithm in the sub-processor of the apparatus then determines the inventory for the consumable test element of a given type as described herein.

Following optical sensing of all sleeves within the drawer of a clinical analyzer apparatus, and when all slots in a sleeve contain the same type test element, then using a processor, inventory of particular test element types is quickly performed by a gripper in the analyzer picking a single consumable test element from test elements each sleeve and reading with a barcode reader or camera system of the type that will be familiar to one having skill in the relevant art, to determine the type of test element loaded in the entire sleeve. However, such methodology does not permit more than one type of test element per sleeve. Since picking every consumable test element in the sleeve, and then in every sleeve within the drawer of the analyzer to determine the consumable type would make inventory function too slow for practical use, the instant invention is directed to a method and container to provide a flexible inventory determination of multiple types of test elements in a single sleeve and for each sleeve loaded into a drawer of a clinical analyzer. This avoids the need to swap out sleeves to introduce test elements of different types.

US 2008/0178653 describes systems and methods for calibrating emission data or other information signals collected during a polymerase chain reaction (PCR), amplification reaction, assay, process, or other reaction. Calibration of multiple detectable materials can be achieved during a single cycle or run, or during a plurality of runs of the reaction. A reading from every well, container, or other support region of a sample support does not have to be taken. Interpolation can be used to determine values for emission data or other information signals that were not taken, or are unknown, using detected emission data, or other detected information signals. By calibrating the detected emission data and the interpolated data, a more accurate reading of emission data or information signal can be obtained.

EP 2295982 describes an apparatus for processing biological material, which comprises: a holder for a vessel for receiving biological material, wherein the vessel comprises at least one vessel part of increased recognisability; a radiation source for irradiating a registration area in the holder, wherein the registration area is associated with the vessel part of increased recognisability; a sensor for measuring the intensity of radiation coming from the registration area; and an evaluating unit which is equipped to register the presence or absence of the vessel by running a comparison between the intensity measured and a defined threshold.

EP 0576291 describes an automated instrument for performing a multitude of clinical assays is disclosed. Receptacles for assay reagents and patient samples, controls and standards are supported on one carousel, and multi-well reaction vessels are supported on another, the two carousels sharing a common axis but driven by independently operating drive mechanisms. The reaction vessel carousel is surrounded by a temperature controlled shell which monitors and controls the temperature of all reaction vessels on the carousel. The two carousels rotate in a programmed manner to bring the various receptacles and vessels in alignment with various workstations which contain elements such as wash tubes, liquid transfer tubes, and detection systems, in a sequence which is governed by a programmed controller.

### SUMMARY OF THE INVENTION

The invention is defined by the claims.

The herein described container and method provide considerable time savings and improvements in throughput when used in conjunction with an automated apparatus, as the inventory function makes possible random access to a greater number of types of test elements loaded within a single sleeve.

These and other features and advantages will become readily apparent from the following Detailed Description, which should be read in conjunction with the accompanying drawings.

### BRIEF DESCRIPTION OF THE DRAWING

FIGS. 1 and 2 are front views of a pair of prior art immunodiagnostic test elements;
FIG. 3 is a partial top perspective view of a prior art immunodiagnostic testing apparatus;
FIG. 4 is a simplified front view of the testing apparatus of Fig. 3;
FIG. 5 is a simplified top perspective view of a prior art immunodiagnostic testing apparatus showing an open drawer;
Fig. 6 is a schematic view of a sleeve.
Fig. 7 is a schematic view of a slide tray showing sleeves with 20 compartments containing two test elements each placed in three lanes.
Fig. 8 is a plan view of immunodiagnostic test elements arranged in a sleeve with 20 compartments containing three types of test element, one for each of three types of clinical immunohematologic tests, with test elements of different types of clinical immunohematologic tests separated by a gap of at least one empty slot;
FIG. 9 is a partial side elevational view of the piercing assembly of the prior art immunodiagnostic testing apparatus of Fig. 3
FIG. 10 depicts a top perspective view of a test element bearing a foil wrap closing the top side of the test element.

### DETAILED DESCRIPTION

The following discussion relates to certain exemplary embodiments of a method for holding multiple types of clinical immunodiagnostic, for instance, immunohematologic test elements such as cards or cassettes within single containers such as boxes or sleeves, and allowing random access to any such card or cassette in any container while permitting fast determination of card/cassette type inventory in all sleeves. It will be readily apparent to those of skill in the field that the inventive concepts described herein also relate to literally any other form of clinical analyzer that supports the functionality of multiple containers such as sleeves, racks or supports with positioning guides, containing test elements. In addition, certain terms are used throughout this discussion in an effort to provide a frame of reference with regard to the accompanying drawings. These terms should not be regarded as limiting, except where so specifically indicated.

For purposes of background, Figs. 1 and 2 illustrate a pair of prior art immunodiagnostic test elements. More specifically, Fig. 1 depicts a gel card 20 while Fig. 2 depicts a glass bead cassette 30. Each of the test elements 20, 30 include a number of common structural features. That is, each test element 20, 30 commonly includes a support member 26 in the form of a planar substrate having a top side 27 and a bottom side 28, wherein the substrate supports a plurality of microtubes or test columns 34. The microtubes 34 are made from a transparent material and are further defines by an upper portion 37 having an open top opening, an inwardly tapering transition portion 39 and a lower portion 41. A predetermined quantity of an inert material 38, 42, is contained within the lower portion 41 of each test column 34, as typically provided by the manufacturer. In the instance of the gel card 20, the inert material 38 is a gel material, such as Sephacryl or other suitable material, while in the instance of the bead cassette 30, the inert material 42 is defined by a matrix of glass or other bead material. Each of the inert material 38, 42 is typically defined by a plurality of particles having a diameter of between about 10 and 100 microns. Typically, the inert material 38, 42 contained in each microtube 34 is further coated with an antibody or provided with a carrier-bound antigen or antibody, such as anti-A, also typically provided by the manufacturer, thereby defining an aqueous medium. At least fifteen (15) types of test elements are available, each for different immunological tests. A pierceable wrap conveniently comprising a foil wrap 50 provided at the top side 27 of each test element 20, 30 covers and seals the microtubes 34 in order to protect the contents and also to prevent dehydration or degrading thereof. Further advantages of this wrap in the practice of the instant invention are discussed hereinbelow.

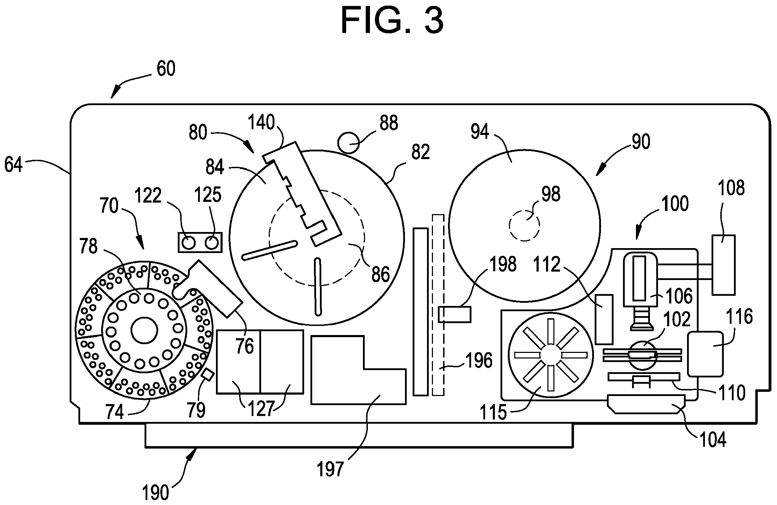

Now with further reference to the accompanying Figures, it is described how the foregoing immunodiagnostic test elements 20, 30 can be used in an automated testing apparatus 60, such as that shown in Figs. 3-5. Those skilled in the art of clinical laboratory blood analysis will understand the following description as exemplary of a clinical blood analysis apparatus. In brief, the testing apparatus 60 retains a number of components including a reagent and sample supply 70, an incubator station 80, a centrifuge 90, an analysis station 100, and a drawer assembly 190, each shown in Fig. 3.

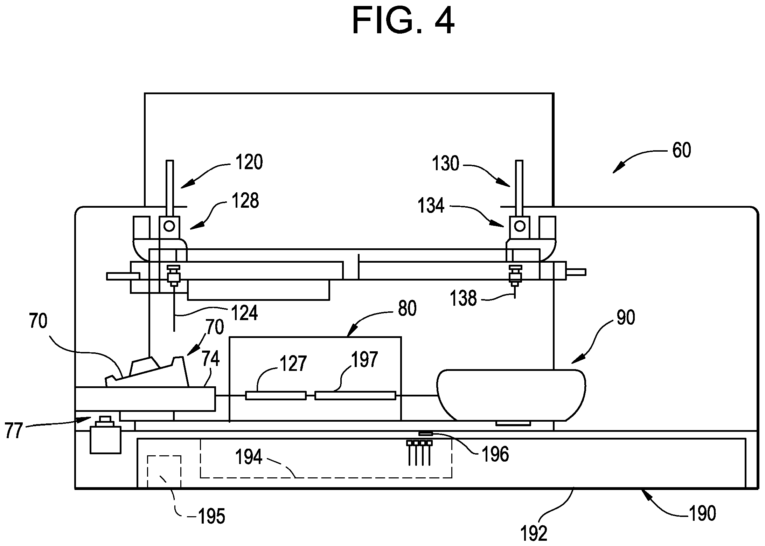

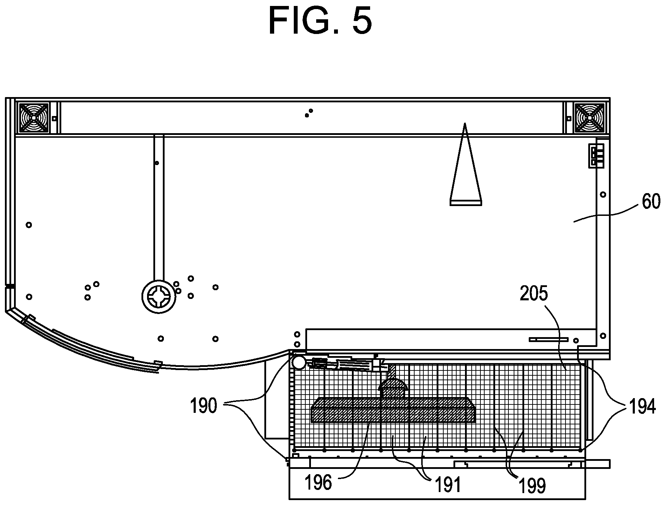

More particularly, the sample and reagent supply 70 of this apparatus 60 includes a sample rack 74 as well as a reagent rack 78, each of which contain bottles or vials of patient sample and reagent, respectively. The supply is constructed as a rotor that is rotatable about a center axis by means of a drive mechanism that includes a motor 77, Fig.4, wherein a bar code reader 79, Fig. 3, is further provided in relation to the supply 70 as well as a tube hold-down assembly 76 disposed over a portion thereof. The incubator station 80 includes a cassette rack 82 that further includes respective first and second sections 84, 86, as well as a drive mechanism that includes a motor 88. The centrifuge 90 includes a rotor 94 and a motor 98. The analysis station 100 includes holding means 102, illumination means 104, an imaging subsystem 106, a processing subsystem 108, a transport subsystem 110, a storage rack 115, a bar code reader 112, and a waste receptacle 116. The drawer assembly 190, Figs.4 & 5, includes a drawer 192, a slide tray 194 which holds a number of sleeves 193, a motor 195, Fig. 4, a sensor bar 196, also shown in Fig. 5, a bar code reader 198, Fig. 4, and a holding area 197. A transport assembly 130, Fig. 4, of the testing apparatus 60 includes a robot arm 134, and a gripper 138. Finally, a pipette assembly 120, Fig. 4, includes a pipette 124 attached to a robot arm 128, this assembly further including shallow and deep wash areas 122,125, as well as cell dilution packs 127.

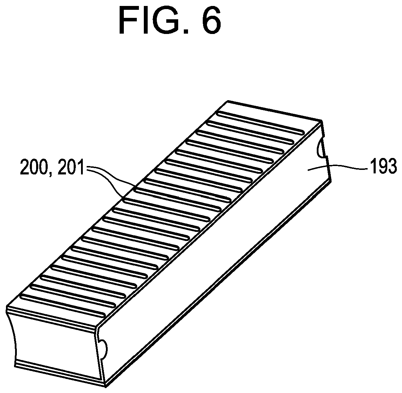

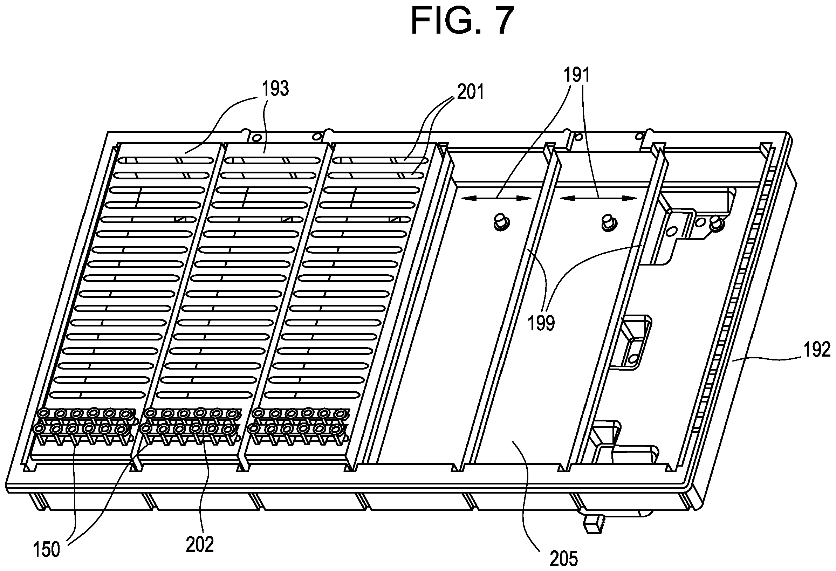

A plurality of test elements 20, 30, such as those previously described according to either Figs. 1 or 2, may be supplied by the manufacturer supported in sleeves 193, Fig. 6, containing compartments or slots 200 designed to accommodate the size and shape of individual test elements. Such sleeves are commonly made of paperboard or cardboard but can be made of any suitable material. The sleeves as commonly supplied contain twenty (20) immunohematologic test elements of a single type, such test elements positioned upright such that the foil wrap on the top of the test element is clearly visible at the top side. The sleeves 193 fit snugly in lanes 191, defined by the right and left sides of the drawer and by rails 199 positioned and affixed to the sides of the drawer (Fig. 7). Test elements 20, 30 are received from the manufacturer in such sleeves which are placed into the lanes 191 of the slide tray of an analyzer drawer in desired numbers up to the capacity of the drawer and ready for use in such immunohematologic tests as ordered by the physician.

In the prior art, only one type of immunohematologic test element card/cassette could be loaded into a given sleeve as there was no functionality of inventorying and choosing a specific test element type from within a single sleeve. In the instant invention, more than one type of test element may conveniently be loaded into a single sleeve and multiple such sleeves may be loaded into the drawer of a clinical analyzer. To do so, the operator removes multiple test elements from a given sleeve as supplied by the manufacturer, and inserts test elements of a different type therefor, grouping all the test elements of a single immunohematologic test type into consecutive slots within the sleeve while leaving at least one slot (thereby forming a gap) empty between the two (or more) types of immunohematologic test elements. It is therefore to be understood that when the test element capacity of a sleeve is x, and when more than one type of test element is to be loaded into a sleeve, the number of test elements so loaded will be not greater than x-1, and test elements may be loaded starting at slot number 1 (placement as shown in Fig. 7). The operator will thus load test element(s) of another type of immunohematologic test into the same sleeve while leaving at least one slot empty between the two types of immunohematologic test elements. The empty slot(s) are location(s) where one or more test element(s) could otherwise be located, and functions as a detectable gap for the optical sensing bar 196, Figs. 4 & 5, of the apparatus. This loading of multiple types of test elements into single sleeves can be done for all sleeves in the drawer of a clinical analyzer. The detectable gap is sensed by sensor through detection of the reflective difference of the presence or absence of a consumable test element having a top side protective cover that has a reflective capacity measurably different from a slot containing no test element; the presence or absence of the test element thus detected by this reflective difference. In a preferred embodiment such top side protective cover is a foil wrap and the sensor can be for example an optical sensor such as an optical proximity sensor. Software in the sub-processor thus determines the inventory for the consumable test element of a given type. This aforementioned sleeve-loading continues for the multiple types of test elements as desired up to the capacity of the sleeve, and is repeated for all sleeves as desired, and up to the full capacity of the slide tray 194 within the drawer 192 at the CCLA of the apparatus 60, with at least one empty slot between each group of test elements of the same type within each sleeve. Therefore the invention provides for sensing of the multiple types of test elements for all groups of test elements within all containers in the drawer of the clinical analyzer.

In the embodiment wherein test elements are contained in sleeves, and once the operator has loaded the test elements 20, 30 of the various types as desired into the sleeves 193, Fig. 5 & 6, and has left at least one empty slot 200 that serves as the detectable gap therebetween, the operator loads the sleeves into the lanes 191 of the slide tray 194 at the card/cassette loading area (CCLA), and closes the drawer 192. Upon any closure of drawer 192, whether due to loading of new sleeves or arranging or adding test elements within or to sleeves, for example each time the contents of the drawer are accessed and the drawer is thereafter closed, and also upon powering on of the apparatus 60, the sensor bar 196 scans all sleeves within all lanes of the drawer, detecting location of groups of test elements within a given sleeve and, where so loaded by the operator, separated from another of test elements by at least one empty slot. It will be apparent that a "group" can consist of a minimum of a single test element of a given type and a maximum of x test elements wherein x is the test element capacity of the sleeve. As stated above, when more than one type of test element is loaded into a single sleeve, the groups of test elements will have at least one empty slot therebetween. Those having skill in the art will know of similar means to detect the test elements 20, 30 within the sleeves 193 resident in the slide tray 194, aside from that disclosed herein. The test elements in the invention have on their top side surface a protective wrap or covering of measurably different reflectance than a slot containing no test element. The presence or absence of the test element can be detected by this reflective difference. In particular in a preferred embodiment of the invention the optical sensor bar 196 communicates with the processing subsystem 108 the difference between the reflectivity of the foil wrap and the reflective capacity of the bottom support member of the sleeve which contains no test element i.e., empty slots, or the lack of a test element in a slot, where any may exist and where they exist when the sleeve contains more than one type of test element. Such reflectance differential is measured by the use of a processor for instance by optical sensing and in particular by optical proximity sensing. In the case where the operator has loaded a single sleeve with the same type of test element, the optical sensor 196 will so detect and using an appropriate algorithm the processing subsystem 108 thereby determines that one type of test element is so loaded in a given sleeve. In the case where the optical sensor 196 through proximity sensing detects within a sleeve groups of test elements separated by at least one empty slot, then using an appropriate algorithm the processing subsystem 108 determines that more than one different type of test element is present in a single sleeve 193. When the optical sensor bar 196 has detected the arrangement and presence or absence of test elements in the slots, and this has been done for all sleeves, the inventory function is complete and the arrangement of test elements is stored in the processing subsystem 108. As stated, this inventory function for all sleeves proceeds after each closure of the drawer 192 and after each power-on of the apparatus.

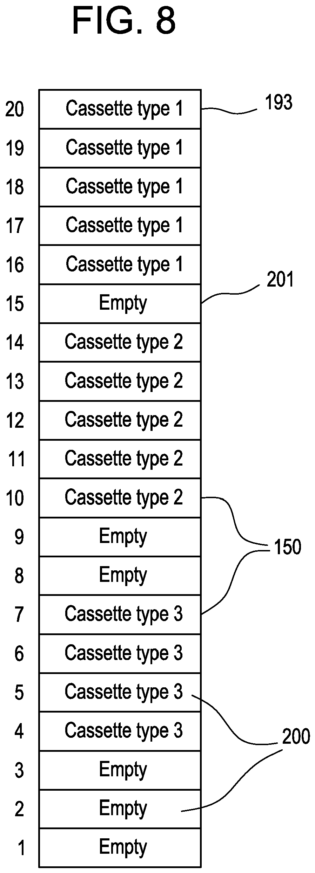

When the optical sensor bar has completed scanning and the results are stored in the processing subsystem, a software algorithm instructs the gripper arm 138, Fig. 4 of the holding means 102, Fig. 3, to grip the first test element of each group in a sleeve. The first test element of a group is the test element in any group closest to an operator standing at the front side of the apparatus 60, and are thus numbered 1-20 in Fig. 8. With reference to Fig. 8, test element position number ascends counting from front to back of the apparatus. With reference to Fig. 8, the first test element the gripper will pick is that test element in the number 4 position. The gripper arm 138 places that test element before illumination means 104 whereby the barcode on the single test element is read by the imaging subsystem 106. The type of test element 20, 30 in that entire group is thereby determined, along with other barcode information on the test element which, as stated above, can include the particular immunohematologic test type, shelf expiration, lot number, and the sequence of that test element within a given lot, among any other indicating information contained in the manufacturer's barcode. This information is then made visible to the operator on the Graphical User Interface or GUI. The information can include for example whether a particular scanned test element is expired or recalled, alerting the operator to deny usage of that card and automatically transport that card to the waste receptacle 116.

The gripper arm, having thus transported the first test element in a first group of test elements and returned that test element to its slot, proceeds to the next group of test elements in the sleeve that are separated by at least one empty slot, as previously detected by proximity sensing by the optical sensor 196 as a detectable gap and stored in the processing subsystem. This information is employed by the processing subsystem to advance the gripper arm to the next group of test elements separated from another group of test elements separated by at least one empty slot, where this configuration may exist in any sleeve. With reference again to Fig. 8, the gripper will then pick the test element is slot numbered 10, and place it before the barcode reader, which reads the barcode information prior to the gripper arm returning the test element to position 10. This activity continues routinely for all groups of test elements within each sleeve loaded into a lane in the drawer of the clinical analyzer, allowing for complete inventorying of the test element contents of each and every sleeve resident in the drawer assembly 190 of the apparatus 60. The inventory function for the various types of test elements within the sleeve and within the drawer of the clinical analyzer is thus achieved and the result of the inventorying function is displayed on the GUI for the operator. Depending on the contents of the sleeves and the test element required for a given test ordered by the physician, the operator may open the drawer and load appropriate type(s) of test element(s) into the one or more sleeves. Where the processing subsystem 108 includes a database or is connected remotely to a Laboratory Information System (LIS) replacement test elements are automatically ordered from a manufacturer or requisitioned for example from another location within a hospital or laboratory as they are used by the apparatus and/or ordered via automated functionality by physicians.

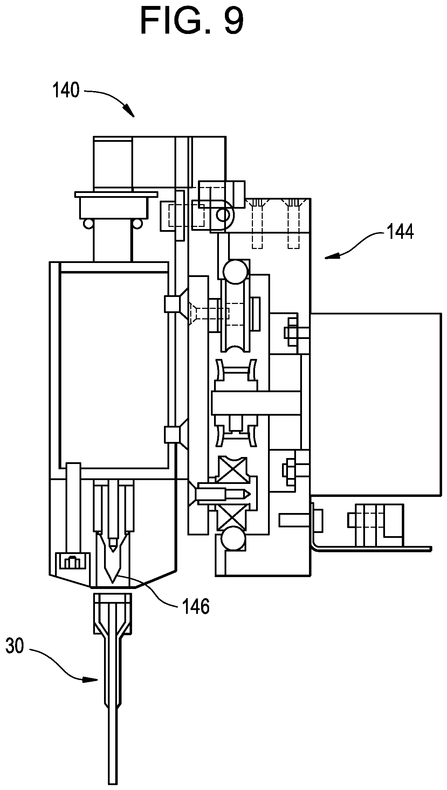

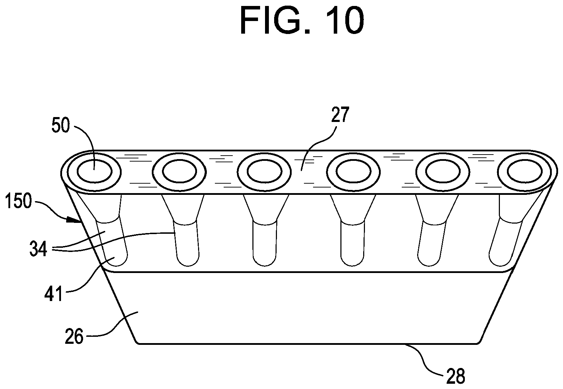

Once the inventorying function including the test element(s)' identification by barcode reader is complete, and the operator calls for an immunohematologic test, the gripper loads an appropriate test element depending on the test to be conducted into the cassette rack 82 of the incubator 80. A piercing assembly 140, Fig. 9, is disposed above the first and second sections 84, 86 of the cassette rack 82 of the incubator 80 and includes a support subassembly 144 that includes a slide support 145, Fig. 9 (not labeled), having a plurality of puncture needles (not shown) that are reciprocably movable, such as by means of solenoids (not shown). The pipette 124 of the pipette assembly 120 is used to aspirate sample from the sample rack 65, while the piercing assembly 140, Fig. 9, is used to puncture the top side protective sealing cover of the test element, such top side cover being for example a foil wrap above each of the microtubes of the then-incubated test elements 20, 30, Fig. 10. Once the puncturing step has been completed as shown by the test elements, the pipette 124 can then be used to dispense a predetermined quantity of patient sample (and possibly additional reagents) from the sample and reagent supply 70 into each of the test columns 34, Fig. 1 & 2, wherein the mixture can be suitably incubated. The incubator 80, as driven by the motor 88, is used to incubate patient sample added to each of the test columns from one of the vials of the sample rack 65, the incubator further including an assembly 76 that holds down the sample and reagent vials.

One having skill in the art will understand that alternative embodiments to the sleeve may include use of a container such as a rack, said rack designed to hold multiple test elements in the appropriate orientation wherein there is left at least one open space in the rack between the test elements. In a further embodiment, the floor 205 of the slide tray 194 may have guides or dividers to support the individual test elements themselves in the appropriate orientation, and wherein the operator would in like fashion leave at least one open space or slot between the types of test elements.

Following incubation and in the described testing apparatus 60, the test elements 20, 30 are removed from the incubator 80 by means of the transport assembly 130 to the centrifuge 90 wherein the test elements 30 are then spun down, thereby accelerating an agglutination reaction as red blood cells are clumped together in the presence of coated reagents. The plurality of beads disposed in each column of the test element 30 includes particles having diameters ranging between about 10 and 100 microns, providing a matrix for the red blood cells, but not the heavier formed agglutinates to pass through by filtering. The resulting reaction can be imaged within the analysis station 100 of the apparatus 60 by means of the illumination assembly 104 and imaging subsystem 106, the latter being connected to the processing subsystem 108 having machine vision for grading of the reaction. Additional details concerning the foregoing testing apparatus 60 are provided in commonly-assigned U.S. Patent No. 5,578,269, to Yaremko et al.

As has been discussed in detail hereinabove, the functionality disclosed permits the apparatus 60 to quickly scan inventory of various test element types by reading a single test element from a group rather than reading test elements individually, thus supporting multiple types of test elements within a single sleeve and random access to each test element within the multiple number of sleeves within a drawer of a clinical analyzer, and thereby providing an efficient inventory of test element in an apparatus.

### PARTS LIST FOR FIGS. 1-13

- 20: gel card
- 26: support member (planar substrate)
- 27: top side
- 28: bottom side
- 30: bead cassette
- 34: microtubes (test column)
- 37: upper portion
- 38: gel material
- 39: inwardly tapering transitional portion
- 41: lower portion
- 42: bead matrix
- 50: foil wrap
- 54: label
- 55: bar code
- 58: panel
- 60: automated testing apparatus
- 64: frame
- 70: sample and reagent supply
- 74: sample rack
- 76: tube hold-down assembly
- 77: drive means
- 78: reagent rack
- 79: bar code reader
- 80: incubator station
- 82: cassette rack
- 84: first section
- 86: second section
- 88: motor
- 90: centrifuge
- 94: rotor
- 98: motor
- 100: analysis station
- 102: holding means
- 104: illumination means
- 106: imaging subsystem
- 108: processing subsystem
- 110: transport subsystem
- 112: bar code reader
- 115: storage rack
- 116: waste receptacle
- 120: pipette assembly
- 122: shallow wash area
- 124: pipette
- 125: deep wash area
- 127: cell dilution racks
- 128: robot arm
- 130: transport assembly
- 134: robot arm
- 138: gripper
- 140: piercing assembly
- 144: support subassembly
- 146: piercing needles
- 150: test element
- 154: weakened or pre-stressed portions
- 170: punch
- 176: punch head
- 180: metering tip member
- 181: direction
- 182: cylindrical body
- 183: sample
- 184: upper tip opening
- 186: lower tip opening
- 188: interior
- 189: metering mechanism
- 190: drawer assembly
- 191: lane
- 192: drawer
- 193: sleeve
- 194: slide tray
- 195: motor
- 196: sensor bar
- 197: holding area
- 198: bar code reader
- 199: rail
- 200: slots in a sleeve
- 201: empty slot
- 202: foil wrap at top side of test element
- 205: floor of slide tray

## Claims

1. A method of determining an inventory of test elements (20, 30) of multiple types stored in a clinical analyzer (60) comprising:
positioning groups of test elements in a container (193) disposed in a drawer (192) of the clinical analyzer,
wherein the container is a sleeve, a rack or support with positioning guides for test elements and in which each test element is independently accessible;
further wherein the container comprises slots (200) for holding the test elements, each of the test elements comprising an immunohematology test card (20) or cassette (30) and a top side surface (27), and each having, on its top side surface, a protective sealing cover,
wherein the positioning comprises positioning all the test elements of a single type into consecutive slots (200) of the container and providing a gap (201) between each group of test elements by leaving at least one slot empty between each group of test elements,
wherein the protective sealing cover is of measurably different reflectance than the gap,
using an optical sensor bar (196) disposed within the clinical analyzer, scanning the test elements positioned within the drawer upon closure of the drawer and also upon powering on of the clinical analyzer,
wherein the scanning comprises detecting locations of the groups of test elements within the container containing multiple groups of test elements, wherein the gap is capable of being detected by the optical sensor bar by detection of the reflective difference between the protective sealing cover and the gap;
determining the type of test element (20, 30) in an entire group, after the gaps have been detected, by picking up the first test element of each group with a gripper arm (138) and placing it before illumination means (104), whereby a bar code affixed on the single test element is read by the imaging subsystem (106), whereby the bar code includes the information about the type of test element;
by using a processing subsystem (108) generating inventory data for the test elements of each type, based on the test element capacity of the container and the detection of at least one gap by the optical sensor bar;
using said data generated by the processing subsystem (108) to provide an inventory of the multiple test elements in the clinical analyzer.

2. The method of claim 1, wherein the sensing is optical proximity sensing, and wherein the result of the optical proximity sensing is stored in the processor.

3. The method of claim 2, wherein said generating data step includes performing an algorithm that determines a change in number of test elements (20, 30) in the group in the container (193) from previously stored data in the number of test elements in the group in the container.

4. The method of claim 3 wherein the protective sealing cover is a foil wrap.

5. The method according to claim 1, further comprising:
using a processor, retrieving a previous indication of the number of test elements (20, 30) in the group in the container (193);
determining a change in the number of test elements in the group in the container to a new number of test elements in the group in the container in the step of generating;
associating the change in the number of test elements in the group in the container to a usage indication; and
storing the association.

6. The method of claim 5, further comprising notifying a user with an indication of the change in the number of test elements (20, 30) in the group in the container (193), wherein the indication is selected from the group comprising a visual indication or an audible indication.

7. The method of claim 1, further comprising enabling automated ordering of replacement test elements (20, 30) from a manufacturer following determining a change in the number of test elements in a group in a clinical analyzer (60) as determined during inventorying test elements in the clinical analyzer, using a processor, through connection to laboratory information system or database.

8. The method of claim 1, further comprising enabling automated requisitioning of replacement test elements (20, 30) from a remote location within a hospital or laboratory following determining a change in the number of test elements in a group in a clinical analyzer (60) as determined during inventorying test elements in the clinical analyzer, using a processor, through connection to a laboratory information system or database.

9. A clinical analyzer, comprising an
optical sensor bar (196);
a container (193) configured to hold multiple types of test elements (20, 30) each being arranged together in the container according to its type and a detectable gap between each type of test element, wherein the container is a sleeve, a rack, or a support with positioning guides for test elements and in which each test element is independently accessible, further wherein the container comprises slots (200) for holding the test elements and the detectable gap (201) is at least one slot containing no test elements, each of the test elements comprising an immunohematology test card (20) or cassette (30) and a top side surface (27), and each having, on its top side surface, a protective sealing cover of measurably different reflectance than the gap, further wherein the gap is detected by measuring the reflective difference between the protective sealing cover of the test element and the slot containing no test elements, and wherein the gap is detected by optical sensing by the optical sensor, including optical proximity sensing;
illumination means (104) and an imaging subsystem (106);
a gripper arm (138) configured to pick up a first test element in each group and to place it before illumination means (104) whereby a bar code affixed on the single test element can be read by the imaging subsystem (106), whereby the bar code includes the information about the type of the test element;
and a processing subsystem (108) configured to generate inventory data for the test elements of each type, based on the test element capacity of the container and the detection of at least one gap by the optical sensor bar (196), and to provide an inventory of the multiple test elements in the clinical analyzer.

## Patentansprüche

1. Verfahren zum Bestimmen eines Bestands von Testelementen (20, 30) mehrerer Typen, die in einem klinischen Analysegerät (60) gelagert sind, umfassend:
Positionieren von Gruppen von Testelementen in einem Behälter (193), der in einer Schublade (192) des klinischen Analysegeräts angeordnet ist,
wobei der Behälter eine Hülse, ein Gestell oder ein Träger mit Positionierungsführungen für Testelemente ist und in dem jedes Testelement unabhängig zugänglich ist;
ferner wobei der Behälter Schlitze (200) zum Halten der Testelemente umfasst, wobei jedes der Testelemente eine Immunhämatologie Testkarte (20) oder Kassette (30) und eine obere Seitenfläche (27) umfasst und jedes auf seiner oberen Seitenfläche eine schützende Dichtungsabdeckung aufweist,
wobei das Positionieren das Positionieren aller Testelemente eines einzigen Typs in aufeinanderfolgende Schlitze (200) des Behälters und das Bereitstellen einer Lücke (201) zwischen jeder Gruppe von Testelementen umfasst, indem mindestens ein Schlitz zwischen jeder Gruppe von Testelementen leer gelassen wird,
wobei die schützende Dichtungsabdeckung einen messbar anderen Reflexionsgrad aufweist als die Lücke,
Verwenden einer optischen Sensorleiste (196), die innerhalb des klinischen Analysegeräts angeordnet ist, Scannen der innerhalb der Schublade positionierten Testelemente beim Schließen der Schublade und auch beim Einschalten des klinischen Analysegeräts,
wobei das Scannen das Erfassen von Positionen der Gruppen von Testelementen innerhalb des Behälters umfasst, der mehrere Gruppen von Testelementen enthält, wobei die Lücke von der optischen Sensorleiste durch Erfassen des Reflexionsunterschieds zwischen der schützenden Dichtungsabdeckung und der Lücke erfasst werden kann;
Bestimmen des Typs des Testelements (20, 30) in einer ganzen Gruppe, nachdem die Lücken erkannt wurden, indem das erste Testelement jeder Gruppe mit einem Greifarm (138) aufgenommen und vor eine Beleuchtungsmittel (104) gelegt wird, wobei ein Strichcode, der auf dem einzelnen Testelement angebracht ist, von dem bildgebenden Teilsystem (106) ausgelesen wird, wobei der Strichcode die Informationen über den Typ des Testelements enthält;
durch Verwenden eines Verarbeitungsteilsystems (108), Erzeugen von Bestandsdaten für die Testelemente jedes Typs, basierend auf der Testelementkapazität des Behälters und der Erfassung von mindestens einer Lücke durch die optischen Sensorleiste;
Verwenden der von dem Verarbeitungsteilsystem (108) erzeugten Daten, um einen Bestand der mehreren Testelemente in dem klinischen Analysegerät bereitzustellen.

2. Verfahren nach Anspruch 1, wobei das Abtasten ein optisches Näherungsabtasten ist und wobei das Ergebnis des optischen Näherungsabtastens im Prozessor gespeichert wird.

3. Verfahren nach Anspruch 2, wobei der Datenerzeugungsschritt das Ausführen eines Algorithmus enthält, der eine Änderung der Anzahl von Testelementen (20, 30) in der Gruppe in dem Behälter (193) aus zuvor gespeicherten Daten der Anzahl von Testelementen in der Gruppe in dem Behälter bestimmt.

4. Verfahren nach Anspruch 3, wobei die schützende Dichtungsabdeckung eine Folienhülle ist.

5. Verfahren nach Anspruch 1, ferner umfassend:
Verwenden eines Prozessors, Abrufen einer vorherigen Anzeige der Anzahl von Testelementen (20, 30) in der Gruppe in dem Behälter (193);
Bestimmen einer Änderung der Anzahl von Testelementen in der Gruppe in dem Behälter zu einer neuen Anzahl von Testelementen in der Gruppe in dem Behälter im Schritt des Erzeugens;
Zuordnen der Änderung der Anzahl von Testelementen in der Gruppe in dem Behälter zu einer Verwendungsanzeige; und
Speichern der Zuordnung.

6. Verfahren nach Anspruch 5, ferner umfassend das Benachrichtigen eines Benutzers mit einer Anzeige der Änderung der Anzahl von Testelementen (20, 30) in der Gruppe in dem Behälter (193), wobei die Anzeige aus der Gruppe ausgewählt ist, die eine visuelle Anzeige oder eine akustische Anzeige umfasst.

7. Verfahren nach Anspruch 1, ferner umfassend das Ermöglichen einer automatisierten Bestellung von Ersatztestelementen (20, 30) von einem Hersteller nach dem Bestimmen einer Änderung der Anzahl von Testelementen in einer Gruppe in einem klinischen Analysegerät (60), wie sie bei der Bestandsaufnahme von Testelementen in dem klinischen Analysegerät bestimmt wurde, unter Verwendung eines Prozessors durch Verbindung mit einem Laborinformationssystem oder einer Datenbank.

8. Verfahren nach Anspruch 1, ferner umfassend das Ermöglichen einer automatisierten Anforderung von Ersatztestelementen (20, 30) von einem entfernten Ort innerhalb eines Krankenhauses oder Labors nach dem Bestimmen einer Änderung der Anzahl von Testelementen in einer Gruppe in einem klinischen Analysegerät (60), wie sie bei der Bestandsaufnahme von Testelementen in dem klinischen Analysegerät bestimmt wurde, unter Verwendung eines Prozessors, durch Verbindung mit einem Laborinformationssystem oder einer Datenbank.

9. Klinisches Analysegerät, umfassend eine optische Sensorleiste (196);
einen Behälter (193), der so konfiguriert ist, dass er mehrere Typen von Testelementen (20, 30) hält, die jeweils zusammen in dem Behälter gemäß ihrem Typ und mit einer erfassbaren Lücke zwischen jedem Typ von Testelement angeordnet sind, wobei der Behälter eine Hülse, ein Gestell oder ein Träger mit Positionierungsführungen für Testelemente ist und in dem jedes Testelement unabhängig zugänglich ist, wobei ferner der Behälter Schlitze (200) zum Halten der Testelemente umfasst und die erfassbare Lücke (201) mindestens ein Schlitz ist, der keine Testelemente enthält, wobei jedes der Testelemente eine Immunhämatologie Testkarte (20) oder Kassette (30) und eine obere Seitenfläche (27) umfasst und jedes auf seiner oberen Seitenfläche eine schützende Dichtungsabdeckung mit messbar unterschiedlichem Reflexionsvermögen als die Lücke aufweist, ferner wobei die Lücke durch Messen des Reflexionsunterschieds zwischen der schützenden Dichtungsabdeckung des Testelements und dem Schlitz, der keine Testelemente enthält, erfasst wird, und wobei die Lücke durch optisches Erfassen durch den optischen Sensor erfasst wird, der ein optisches Näherungsabtasten enthält;
Beleuchtungsmittel (104) und ein bildgebendes Teilsystem (106);
einen Greifarm (138), der so konfiguriert ist, dass er ein erstes Testelement in jeder Gruppe aufnimmt und es vor ein Beleuchtungsmittel (104) legt, wobei ein Strichcode, der auf dem einzelnen Testelement angebracht ist, durch das bildgebende Teilsystem (106) ausgelesen werden kann, wobei der Strichcode die Informationen über den Typ des Testelements enthält;
und ein Verarbeitungsteilsystem (108), das so konfiguriert ist, dass es Bestandsdaten für die Testelemente jedes Typs basierend auf der Testelementkapazität des Behälters und der Erfassung mindestens einer Lücke durch die optische Sensorleiste (196) erzeugt, und einen Bestand der mehreren Testelemente im klinischen Analysegerät bereitstellt.

## Revendications

1. Procédé de détermination d'un inventaire d'éléments de test (20, 30) de types multiples stockés dans un analyseur clinique (60) comprenant :
le positionnement de groupes d'éléments de test dans un conteneur (193) disposé dans un tiroir (192) de l'analyseur clinique,
le conteneur étant un rodet, un portoir ou un support avec des guides de positionnement pour des éléments de test et chaque élément de test étant accessible indépendamment ;
le conteneur comprenant en outre des fentes (200) pour contenir les éléments de test, chacun des éléments de test comprenant une carte de test immunohématologique (20) ou une cassette (30) et une surface latérale supérieure (27), et chacun ayant, sur sa face supérieure surface, un couvercle d'étanchéité protecteur,
le positionnement comprenant le positionnement de tous les éléments de test d'un même type dans des fentes (200) consécutives du conteneur et la fourniture d'un espace (201) entre chaque groupe d'éléments de test en laissant au moins une fente vide entre chaque groupe d'éléments de test,
le couvercle d'étanchéité protecteur ayant une réflectance mesurable différente de celle de l'espace,
à l'aide d'une barre de capteur optique (196) disposée à l'intérieur de l'analyseur clinique, le balayage des éléments de test positionnés à l'intérieur du tiroir lors de la fermeture du tiroir et également lors de la mise sous tension de l'analyseur clinique,
le balayage comprenant la détection des emplacements des groupes d'éléments de test à l'intérieur du conteneur contenant de multiples groupes d'éléments de test, l'espace étant apte à être détecté par la barre de capteur optique par détection de la différence de réflexion entre le couvercle d'étanchéité protecteur et l'espace ;
la détermination du type d'élément de test (20, 30) dans un groupe entier, après détection des écarts, en saisissant le premier élément de test de chaque groupe avec un bras de préhension (138) et en le plaçant devant un moyen d'éclairage (104), grâce à quoi un code à barres apposé sur l'élément de test unique est lu par le sous-système d'imagerie (106), grâce à quoi le code à barres comprend les informations concernant le type d'élément de test ;
à l'aide d'un sous-système de traitement (108), la génération de données d'inventaire pour les éléments de test de chaque type, sur la base de la capacité d'éléments de test du conteneur et de la détection d'au moins un espace par la barre de capteur optique ;
l'utilisation desdites données générées par le sous-système de traitement (108) pour fournir un inventaire des multiples éléments de test dans l'analyseur clinique.

2. Procédé selon la revendication 1, dans lequel la détection est une détection de proximité optique, et dans lequel le résultat de la détection de proximité optique est stocké dans le processeur.

3. Procédé selon la revendication 2, dans lequel ladite étape de génération de données comporte l'exécution d'un algorithme qui détermine un changement du nombre d'éléments de test (20, 30) dans le groupe dans le conteneur (193) à partir de données précédemment stockées dans le nombre d'éléments de test dans le groupe dans le conteneur.

4. Procédé selon la revendication 3, dans lequel le couvercle d'étanchéité protecteur est une feuille d'aluminium.

5. Procédé selon la revendication 1, comprenant en outre :
à l'aide d'un processeur, la récupération d'une indication précédente du nombre d'éléments de test (20, 30) dans le groupe dans le conteneur (193) ;
la détermination d'un changement du nombre d'éléments de test dans le groupe dans le conteneur en un nouveau nombre d'éléments de test dans le groupe dans le conteneur dans l'étape de génération ;
l'association du changement du nombre d'éléments de test dans le groupe dans le conteneur à une indication d'utilisation ; et
le stockage de l'association.

6. Procédé selon la revendication 5, comprenant en outre la notification à un utilisateur d'une indication du changement du nombre d'éléments de test (20, 30) dans le groupe dans le conteneur (193), l'indication étant sélectionnée dans le groupe comprenant une indication visuelle ou une indication sonore.

7. Procédé selon la revendication 1, comprenant en outre l'activation de la commande automatisée d'éléments de test de remplacement (20, 30) auprès d'un fabricant suite à la détermination d'un changement du nombre d'éléments de test dans un groupe dans un analyseur clinique (60) tel que déterminé pendant l'inventaire des éléments de test dans l'analyseur clinique, à l'aide d'un processeur, par connexion au système d'informations ou à la base de données du laboratoire.

8. Procédé selon la revendication 1, comprenant en outre l'activation de la demande automatisée d'éléments de test de remplacement (20, 30) à partir d'un emplacement distant dans un hôpital ou un laboratoire suite à la détermination d'un changement du nombre d'éléments de test dans un groupe dans un analyseur clinique (60) comme déterminé lors de l'inventaire des éléments de test dans l'analyseur clinique, à l'aide d'un processeur, par connexion à un système d'informations ou à une base de données de laboratoire.

9. Analyseur clinique, comprenant une barre de détection optique (196) ;
un conteneur (193) conçu pour contenir plusieurs types d'éléments de test (20, 30) chacun étant agencé ensemble dans le conteneur selon son type et un espace détectable entre chaque type d'élément de test, le conteneur étant un rodet, un portoir ou un support avec des guides de positionnement pour les éléments de test et chaque élément de test étant accessible indépendamment, le conteneur comprenant en outre des fentes (200) pour maintenir les éléments de test et l'espace détectable (201) étant au moins une fente ne contenant aucun élément de test, chacun des éléments de test comprenant une carte de test immunohématologique (20) ou une cassette (30) et une surface latérale supérieure (27), et chacun ayant, sur sa surface latérale supérieure, un couvercle d'étanchéité protecteur d'une réflectance mesurable différente de celle de l'espace, en outre l'espace étant détecté en mesurant la différence de réflexion entre le couvercle d'étanchéité protecteur de l'élément de test et la fente ne contenant aucun élément de test, et l'espace étant détecté par détection optique par le capteur optique, y compris la détection optique de proximité ;
un moyen d'éclairage (104) et un sous-système d'imagerie (106) ;
un bras de préhension (138) conçu pour saisir un premier élément de test dans chaque groupe et pour le placer devant un moyen d'éclairage (104) de sorte qu'un code à barres apposé sur l'élément de test unique peut être lu par le sous-système d'imagerie (106), de sorte que le code à barres comporte les informations sur le type de l'élément de test ;
et un sous-système de traitement (108) configuré pour générer des données d'inventaire pour les éléments de test de chaque type, sur la base de la capacité d'éléments de test du conteneur et de la détection d'au moins un espace par la barre de détection optique (196), et pour fournir un inventaire des multiples éléments de test dans l'analyseur clinique.
